# EUROPÄISCHE PATENTANMELDUNG

(11) **EP 1 500 640 A1**
(43) Veröffentlichungstag der Anmeldung: **26.01.2005**
(21) Anmeldenummer: 04025492.2
(22) Anmeldetag: 24.08.1999
(51) Int. Cl.: C07C 17/38, C07C 17/389, C07C 19/08, C01B 7/07, C01B 7/19

(54) **Reinigung von 1,1,1,3,3-Pentafluorbutan**

(30) Priorität: 03.09.1998 DE 19840099
(62) Teilanmeldung aus: 99968662.9
(71) Anmelder: Solvay Fluor und Derivate GmbH, 30173 Hannover (DE)
(72) Erfinder: Brosch, Carsten, 30926 Seelze (DE); Gress, Heinz, 30457 Hannover (DE); Rieland, Matthias, 30559 Hannover (DE)
(74) Vertreter: Fischer, Reiner, Dr.

(57) **Zusammenfassung**

Beschrieben wird die Herstellung von gereinigtem 1,1,1,3,3-Pentafluorbutan (HFC-365mfc) mit einem verringerten Gehalt an Chlorwasserstoff, Fluorwasserstoff bzw. ungesättigten Verunreinigungen. Hierzu behandelt man das zu reinigende Material in der Flüssigphase mit einem festen, anorganischen Sorptionsmittel. Der Gehalt an Fluorwasserstoff und Chlorwasserstoff kann auf unter 1 ppm, der Gehalt an ungesättigten (Chlor)-Fluor-Verbindungen auf unter 20 ppm und der Gehalt an ungesättigten C2-Verbindungen auf unter 10 ppm gedrückt werden. Es wurde weiterhin gefunden, dass Fluortrichlorethylen als Kontrollsubstanz beim Monitoring der Reinigung von 1,1,1,3,3-Pentafluorbutan verwendet werden kann.

## Beschreibung

Die Erfindung bezieht sich auf ein Verfahren zur Herstellung von gereinigtem 1,1,1,3,3-Pentafluorbutan (HFC-365mfc).

1,1,1,3,3-Pentafluorbutan wird beispielsweise als Treibmittel für die Herstellung von geschäumten Kunststoffen verwendet. Es kann beispielsweise aus der entsprechenden Pentachlorbutanverbindung, Fluorwasserstoff und einem Fluorierungskatalysator hergestellt werden. Das auf diese Weise hergestellte 1,1,1,3,3-Pentafluorbutan kann Chlorwasserstoff, Fluorwasserstoff bzw. ungesättigte Kohlenstoffverbindungen enthalten, welche aus der Fluorierungsreaktion oder dem Einsatzmaterial entstammen. Es ist wünschenswert, aus dem verunreinigten Produkt ein gereinigtes Produkt herzustellen. Diese Aufgabe wird durch das Verfahren der vorliegenden Erfindung gelöst.

Das erfindungsgemäße Verfahren sieht vor, dass man zur Herstellung von gereinigtem 1,1,1,3,3-Pentafluorbutan mit einem verringerten Gehalt an HCl, HF und/oder an ungesättigten Verunreinigungen aus rohem 1,1,1,3,3-Pentafluorbuten, wobei man das rohe 1,1,1,3,3-Pentafluorbutan in der Flüssigphase mit einem festen, anorganischen Sorptionsmittel oder zweiatomigen, an C-C-Mehrfachbindungen addierenden Molekülen behandelt und das behandelte 1,1,1,3,3-Pentafluorbutan abtrennt.

Druck und Temperatur werden so gewählt, dass man in der Flüssigphase arbeitet. Bevorzugt liegt der Druck bei 1 bis 5 atm (abs).

Mit festen, anorganischen Sorptionsmitteln kann man sowohl die sauren Komponenten als auch die ungesättigten Verunreinigungen abtrennen. Mit den zweiatomigen Molekülen gelingt die Derivatisierung der ungesättigten Verbindungen zu nichttoxischen und/oder destillativ abtrennbaren Verbindungen.

Bevorzugte feste, anorganische Sorptionsmittel sind Aktivkohle und Sorbentien auf Basis von Aluminiumoxid oder Siliciumdioxid. Sie eignen sich besonders gut zur Abtrennung von Chlorwasserstoff und/oder Fluorwasserstoff.

Die Behandlung mit dem Sorptionsmittel wird vorteilhaft bei einer Temperatur von -30 °C bis +100 °C, vorzugsweise 15 bis 25 °C durchgeführt.

Zur Verringerung des Gehaltes an ungesättigten Verbindungen setzt man als bevorzugtes zweiatomiges Molekül Chlorwasserstoff oder elementares Fluor, Chlor oder Wasserstoff ein.

Dabei kann man so vorgehen, dass man zunächst mittels der genannten zweiatomigen Moleküle den Gehalt an ungesättigten Verbindungen verringert und anschließend mittels eines festen anorganischen Sorptionsmittels weitere Verunreinigungen verringert.

Besonders bevorzugt ist es, zur Verringerung des Gehaltes an ungesättigten Verbindungen elementares Fluor, vorzugsweise im Gemisch mit einem Inertgas wie Stickstoff oder Argon einzusetzen. Die Behandlung mit elementarem Fluor (bzw. seinen Gemischen mit Inertgas) führt man zweckmäßig bei einer Temperatur im Bereich von -80 bis +20 °C, vorzugsweise im Bereich von -20 bis -10 °C durch. Bereits bei niedriger Fluorkonzentration, bis hin zu 10 Vol.-%, ist eine gute Wirkung zu beobachten.

Je nach Behandlungsdauer oder der eingesetzten Menge des die Reinigung bewirkenden Mittels kann man die Verunreinigungen mehr oder weniger vollständig abtrennen. So setzt man beispielsweise weniger Fluor ein, als für die Anlagerung an die ungesättigten Verunreinigungen benötigt wird, verbleibt eine entsprechende Menge der Verunreinigung im zu reinigenden Produkt. Dies kann aber durch einfache Handversuche und Produktanalyse ermittelt werden. Vorzugsweise führt man die Behandlung mit elementarem Fluor oder einem Gemisch von Fluor und Inertgas solange durch, bis ein 1,1,1,3,3-Pentafluorbutan erhalten wird, welches einen maximalen Gehalt an ungesättigten Chlor-Fluor-Verbindungen von 20 ppm und ungesättigten C2-Verbindungen von 10 ppm aufweist. Der Gehalt an Chlorwasserstoff und/oder Fluorwasserstoff wird solange durchgeführt, bis maximal je 1 ppm enthalten sind; hierzu verwendet man insbesondere die Behandlung mit amorphem Siliciumdioxid oder Aluminiumoxid.

Hat man die Behandlung des rohen Produktes mit einem zweiatomigen Molekül vorgenommen, kann die Auftrennung des resultierenden Produktes in reines Pentafluorbutan und andere Halogenkohlenwasserstoffe durch fraktionierte Kondensation oder beispielsweise auch durch Destillation erfolgen.

Mittels des erfindungsgemäßen Verfahrens ist es möglich, 1,1,1,3,3-Pentafluorbutan herzustellen, welches einen Gehalt von maximal 1 ppm an HF, 1 ppm an HCl, 10 ppm an ungesättigten (Chlor)-Fluor-Verbindungen und 10 ppm an ungesättigten C2-Verbindungen aufweist. Derart hochreines Pentafluorbutan ist neu und ebenfalls Gegenstand der vorliegenden Erfindung.

Das erfindungsgemäße Verfahren eignet sich hervorragend zur Reinigung von 1,1,1,3,3-Pentafluorbutan, welches durch Fluortrichlorethylen (als einziger Verunreinigung oder Bestandteil von Verunreinigungen) kontaminiert ist. Es wurde festgestellt, dass Fluortrichlorethylen besonders schwer abzutrennen ist, da es sehr unreaktiv ist. Mittels des erfindungsgemäßen Verfahrens kann selbst diese Verunreinigung auf einen Gehalt von maximal 20 ppm, ja auf einen Gehalt von weniger als 0,1 ppm verringert werden.

Aufgrund seiner Reaktionsträgheit kann Fluortrichlorethylen als Kontrollsubstanz bei der Überwachung ("beim Monitoring") der Reinigung von 1,1,1,3,3-Pentafluorbutan, welches durch Fluortrichlorethylen und weitere ungesättigte Verbindungen kontaminiert ist, verwendet werden. Es wurde überraschend gefunden, dass lediglich die Abreicherung von Fluortrichlorethylen überwacht werden muss und die gleichzeitige Überwachung der Abreicherung anderer ungesättigter Verbindungen entfallen kann. Wenn Fluortrichlorethylen auf den gewünschten Gehalt herabgedrückt ist, sind andere ungesättigte Verbindungen ebenfalls abgereichert. Die Verwendung von Fluortrichlorethylen als Kontrollsubstanz beim Monitoring der Reinigung von 1,1,1,3,3-Pentafluorbutan, welches Fluortrichlorethylen sowie weitere ungesättigte Verbindungen als Verunreinigungen enthält, ist ebenfalls Gegenstand der Erfindung. Dabei kann das Monitoring mittels GC-MS (SIM-Lauf = Selective Ion Mass) überwacht werden. Die Nachweisgrenze für CFC1111 bei der Bestimmung mittels Gaschromatographie (GC) - thermische Leitfähigkeitsbestimmung (t.c.d., thermal conductivity detection) liegt bei 100 ppm. Mittels GC-MS (g.c. - m.s.) im SIM-Lauf-Modus (SIM run status) liegt die Nachweisgrenze bei 0,1 ppm CFC1111.

Mittels des erfindungsgemäßen Reinigungsverfahrens kann hochreines 1,1,1,3,3-Pentafluorbutan hergestellt werden. Bislang wurden derartige Reinigungsoperationen nur noch bei fluorierten oder vollhalogenierten Verbindungen hoher Stabilität durchgeführt. Insbesondere die Behandlung mit elementarem Fluor liefert ein überraschendes Resultat; denn man hätte statt der Anlagerung an ungesättigte Verbindungen die Substitutionsreaktion am Pentafluorbutan unter Bildung von Hexafluorbutan, Heptafluorbutan oder Perfluorbutan befürchten müssen. Die erfindungsgemäße Verwendung von Fluortrichlorethylen als Kontrollsubstanz beim Monitoring führt zu einer Zeitersparnis, da man sich bei der Aufnahme bzw. der Auswertung von Spektren auf den Bereich konzentrieren kann, in welchem Fluortrichlorethylen registriert wird. Das Arbeiten in der Flüssigphase spart Energie.

Die folgenden Beispiele sollen die Erfindung weiter erläutern, ohne sie in ihrem Umfang einzuschränken. Alle Versuche wurden in der Flüssigphase durchgeführt.

### Beispiel 1:

### Abtrennung ungesättigter Komponenten

a) Eingesetzt wurde HFC365mfc mit 0,22 % (Flächenprozent im Gaschromatogramm) CFC-1111 und 99,4 % HFC365mfc.
al) 84,8 g des Rohproduktes wurden mit 6,8 g Chlor in Gegenwart von 0,2 g FeCl₃ bei 40 °C zur Reaktion gebracht. Das Reaktionsgemisch wurde destilliert. Im Destillat waren noch 0,11 % CFC-1111 nachzuweisen. Die Menge an dieser Verunreinigung war somit halbiert worden.
all) 115,3 g des Rohproduktes wurden über einen Katalysator (4,5 Gew.-% Pd; 0,5 Gew.-% Rh, auf Aktivkohle) mit 0,8 g H₂ im Autoklaven hydriert. Im Reaktionsgemisch waren nur noch 0,03 % CFC-1111 enthalten.
b) Eingesetzt wurde HFC365mfc als Rohprodukt mit 0,16 % (Flächenprozent im Gaschromatogramm) CFC-1111 und 99,68 % HFC365mfc.
bI) 93,6 g des Rohproduktes wurden mit 2,3 g Jod (in Form von KJ/J₂ umgesetzt. Im Reaktionsgemisch waren noch 0,08 % CFC-1111 enthalten.
bII) 195,6 g des Rohproduktes wurden mit 0,72 Litern eines F₂/N₂-Gemisches (10 % F₂) bei -20 °C umgesetzt. Im Reaktionsgemisch war kein CFC-1111 mehr nachweisbar.

### Beispiel 2:

### Abtrennung von CFC-1111 und sauren Komponenten

Eingesetzt wurden 272 kg HFC365mfc mit einem Gehalt an CFC-1111 von ca. 0,2 % (Flächenprozent im GB). Das entspricht ca. 2.000 ppm. Die Reaktion wurde in einem Reaktor mit Umwälzkreislauf durchgeführt. Bei Kühlung auf -12 °C wurden pro Stunde 150 I eines F₂/N₂-Gemisches durchgeleitet (3 Vol.-% F₂). Dauer: 12 Stunden. Danach wurde das Reaktionsgemisch erwärmt und HFC365mfc abdestilliert. Der Gehalt von sauren Bestandteilen (insbesondere HCl und HF) wurde das Destillat mit einem Adsorbens auf SiO₂-Basis kontaktiert. Hierzu wurde als Adsorbens "AF400®" verwendet. Dies ist ein aluminiumfreies, perlförmiges Adsorbens auf SiO₂-Basis mit einem Porendurchmesser von 400 Å (40 nm), Lieferant: Kali-Chemie/Engelhard, Nienburg.

Nach Abtrennen des HFC365mfc vom Adsorbens lag der Gehalt an HCl unterhalb von 1 ppm, der Gehalt an HF ebenfalls unterhalb von 1 ppm.

(Anmerkung: Zur Bestimmung des Gehaltes an CFC-1111 im Produkt wurde der Sim-MS-Status des GC-MS-Gerätes gefahren. Vorab wurde eine Eichkurve erstellt, um den lonenstrom bei 0,1 ppm CFC-1111 und 10 ppm zu bestimmen (mittels hierfür angefertigter Mischungen von HFC365mfc und CFC-1111 der entsprechenden Konzentration). Mittels der Eichkurve wurden dann die beobachteten lonenströme und die zugehörigen Konzentrationen an CFC-1111 korreliert.

## Patentansprüche

1. Verfahren zur Herstellung von gereinigtem 1,1,1,3,3-Pentafluorbutan mit einem verringerten Gehalt an HCl, HF und/oder an ungesättigten Verunreinigungen aus rohem 1,1,1,3,3-Pentafluorbutan, wobei man das rohe 1,1,1,3,3-Pentafluorbutan in der Flüssigphase mit einem festen, anorganischen Sorptionsmittel behandelt und das behandelte 1,1,1,3,3-Pentafluorbutan abtrennt.

2. Verfahren nach Anspruch 1, **dadurch gekennzeichnet, dass** man als festes, anorganisches Sorptionsmittel Aktivkohle oder ein Sorbens auf Basis von Aluminiumoxid oder Siliciumdioxid einsetzt.

3. Verfahren nach Anspruch 2, **dadurch gekennzeichnet, dass** man das Sorptionsmittel zur Abtrennung von HCl und/oder HF einsetzt.

4. Verfahren nach Anspruch 1, **dadurch gekennzeichnet, dass** man die Behandlung mit dem Sorptionsmittel bei einer Temperatur von -30 bis +100, vorzugsweise 15 bis 25 °C durchführt.

5. Verfahren nach Anspruch 1, **dadurch gekennzeichnet, dass** man die Behandlung mit amorphen SiO₂ oder Al₂O₃ solange durchführt, bis der Gehalt an HCl und/oder HF maximal je 1 ppm beträgt.

6. 1,1,1,3,3-Pentafluorbutan, **gekennzeichnet durch** einen Gehalt von maximal 1 ppm an HF, 1 ppm an HCl, 20 ppm an ungesättigten (Chlor)-Fluor-Verbindungen und 10 ppm an ungesättigten C2-Verbindungen.

7. Verwendung von Fluortrichlorethylen als Kontrollsubstanz beim Monitoring der Reinigung von 1,1,1,3,3-Pentafluorbutan, welches Fluortrichlorethylen sowie weitere ungesättigte Verbindungen als Verunreinigungen enthält.
